(19) Europäisches Patentamt
European Patent Office
Office européen des brevets

(11) **EP 2 671 513 A1**

(12) **EUROPEAN PATENT APPLICATION**
published in accordance with Art. 153(4) EPC

(43) Date of publication:
**11.12.2013 Bulletin 2013/50**

(51) Int Cl.:
*A61B 8/12* (2006.01)       *A61B 1/00* (2006.01)
*A61B 1/303* (2006.01)     *A61B 1/307* (2006.01)
*A61B 1/31* (2006.01)

(21) Application number: **12843634.2**

(22) Date of filing: **25.10.2012**

(86) International application number:
**PCT/JP2012/077574**

(87) International publication number:
**WO 2013/062039 (02.05.2013 Gazette 2013/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.10.2011 JP 2011236391**

(71) Applicant: **Olympus Medical Systems Corp. Tokyo 151-0072 (JP)**

(72) Inventors:
• **HASHIGUCHI, Toshihiko Tokyo 151-0072 (JP)**

• **KAWSHIMA, Tomonao Tokyo 151-0072 (JP)**
• **NISHINA, Kenichi Tokyo 151-0072 (JP)**

(74) Representative: **Schicker, Silvia Wuesthoff & Wuesthoff Patent- und Rechtsanwälte Schweigerstrasse 2 81541 München (DE)**

(54) **ULTRASONIC OBSERVATION DEVICE**

(57)    The invention has an object of providing an ultrasound observation apparatus that is easily inserted and for this object the invention includes: an ultrasound transducer 21 that includes an ultrasound transmitting/receiving face 21a that transmits/receives ultrasound; a holding part 22, 23 that fixedly holds the ultrasound transducer; an insertion portion 12 formed in a rigid cylindrical shape in which the holding part is fixedly installed at a distal end; and an insertion assisting instrument 31 that is formed in a rigid rod shape or cylindrical shape, and that is insertedly disposed in a freely insertable/extractable manner in the insertion portion; wherein: the holding part is fixedly installed at the distal end of the insertion portion so as to form a shape that is curved with a predetermined angle in a separating direction with respect to a longitudinal direction from the distal end of the insertion portion; and a curvature angle of the holding part with respect to the longitudinal direction of the insertion portion is set so that, when the insertion assisting instrument is insertedly disposed in the insertion portion, an extension line of a straight line that contacts a distal end face of the insertion assisting instrument contacts the ultrasound transmitting/receiving face.

**FIG.6**

**Description**

Technical Field

This invention relates to an ultrasound observation apparatus that can be inserted transurethrally.

Background Art

The number of patients affected by prostate cancer has been increased in recent years. Conventionally, transrectal biopsy under ultrasound observation has been the predominant technique used to confirm diagnosis of prostate cancer.

In general, at the time of a needle biopsy, the longer a needle path in the tumor tissue is, the greater the amount of tumor cells that can be sampled, and hence the probability of tumor cells being confirmed increases. It is therefore desirable to take a long needle path.

In addition, there is a tendency for prostate cancer to frequently occur at the peripheral zone of the prostate gland. However, in a transrectal biopsy, there is a tendency for a part of the needle path that passes the peripheral zone of the prostate gland to be short. Consequently, there is a limit to improving the probability of discovering prostate cancer cells by transrectal biopsy.

Therefore, performing transurethral biopsy under ultrasound observation can be considered as one technique for sampling tumor cells more reliably. For example, means that transrectally or transurethrally inserts a flexible insertion portion (probe) is disclosed with respect to an apparatus described in Japanese Patent Application Laid-Open Publication No. 2001-37775 and the like.

However, regarding an apparatus disclosed by the above-mentioned Japanese Patent Application Laid-Open Publication No. 2001-37775, etc., only examples using flexible insertion portions are described and there is not any disclosure regarding other means, e.g. transurethral insertion of a rigid insertion portion.

In general, in order to insert the rigid insertion portion into a urethra which is a narrow and occlusive lumen so that a distal end portion of the insertion portion reaches the vicinity of the prostate gland, it is necessary to make an innovation of configuration in an insertion portion shape, in particular a shape of the distal end portion to be easily inserted. Particularly, in a case of the transurethral insertion, since the insertion is performed while spreading a closed urethra, there is a case where a smooth insertion is hard to be performed depending on the shape of the distal end portion.

As shapes of the distal end portion of the insertion portion of the conventional ultrasound observation apparatus, there are ones in approximately planar shapes at distal ends and ones formed to have ring shaped protrusions for attaching balloons (mechanical radial types), etc. However, it is hard to say that any of these conventional ones are appropriate shapes for the insertion while spreading the occluded urethra.

Further, in performing the transurethral insertion, the urethra has a shape feature of bending greatly from the vicinity of the urethral sphincter to the vicinity of the prostate gland. Therefore, it is necessary to make a further innovation of the shape of the distal end portion of the insertion portion in order to pass the distal end of the insertion portion more smoothly at the bent part of the urethra.

The present invention has been made in view of the foregoing points and an object of the present invention is to provide an ultrasound observation apparatus capable of performing transurethral insertion of an insertion portion easily, safely and smoothly by making an innovation of a distal end shape of an insertion portion in an ultrasound observation apparatus that performs transurethral biopsy.

Disclosure of Invention

Means for Solving the Problem

In order to achieve the above object, an ultrasound observation apparatus according to one aspect of the present invention includes: an ultrasound transducer having an ultrasound transmitting/receiving face that transmits/receives ultrasound; a holding part that fixedly holds the ultrasound transducer; an insertion portion formed in a rigid cylindrical shape in which the holding part is fixedly installed at a distal end; and an insertion assisting instrument that is formed in a rigid rod shape or cylindrical shape, and that is insertedly disposed in a freely insertable/extractable manner in the insertion portion; wherein: the holding part is fixedly installed at the distal end of the insertion portion so as to form a shape that is curved with a predetermined angle in a separating direction with respect to a longitudinal direction from the distal end of the insertion portion; and a curvature angle of the holding part with respect to the longitudinal direction of the insertion portion is set so that, when the insertion assisting instrument is insertedly disposed in the insertion portion, an extension line of a straight line that contacts a distal end face of the insertion assisting instrument contacts the ultrasound transmitting/receiving face.

According to the present invention, there is provided an ultrasound observation apparatus capable of performing transurethral insertion of an insertion portion easily, safely and smoothly by making an innovation of a distal end shape of an insertion portion in an ultrasound observation apparatus that performs transurethral biopsy.

Brief Description of the Drawings

Fig. 1 is a perspective view that illustrates an overall configuration of an ultrasound observation apparatus according to a first embodiment of the present invention;

Fig. 2 is an enlarged perspective view of a principal portion that illustrates, in an enlarged manner, an ultrasound probe in a state in which an insertion assisting instrument is mounted thereto in the ultrasound observation apparatus shown in Fig. 1;

Fig. 3 is an enlarged perspective view of a principal portion that illustrates, in an enlarged manner, a vicinity of a distal end portion of the ultrasound probe in the ultrasound observation apparatus shown in Fig. 1;

Fig. 4 is an exposed perspective view of a principal portion that illustrates, in an exposed manner, components of the distal end portion of the ultrasound probe in the ultrasound observation apparatus shown in Fig. 1;

Fig. 5 is an enlarged perspective view of a principal portion that illustrates, in an enlarged manner, a vicinity of a distal end portion of the ultrasound probe in the ultrasound observation apparatus shown in Fig. 1;

Fig. 6 is an enlarged longitudinal sectional view of a principal portion along a longitudinal direction of the ultrasound probe in the ultrasound observation apparatus shown in Fig. 1, that is a sectional view that illustrates the internal configuration in the vicinity of the distal end portion of the ultrasound probe;

Fig. 7 is a plan view when viewing the vicinity of the distal end portion of the ultrasound probe in the ultrasound observation apparatus shown in Fig. 1 from an undersurface side;

Fig. 8 is an enlarged view of a principal portion that shows constituent members taken out from an ultrasound transducer unit arranged at the distal end portion of the ultrasound probe in the ultrasound observation apparatus shown in Fig. 1;

Fig. 9 is an enlarged perspective view of a principal portion that illustrates components, excluding the ultrasound transducer, of the distal end portion of the ultrasound probe in the ultrasound observation apparatus shown in Fig. 1;

Fig. 10 is an enlarged perspective view of a principal portion that illustrates, in an enlarged manner, the vicinity of a proximal end portion of the ultrasound probe in a state in which the insertion assisting instrument is mounted thereto in the ultrasound observation apparatus shown in Fig. 1;

Fig. 11 is an enlarged perspective view of a principal portion that illustrates, in a further enlarged manner, the vicinity of the proximal end portion of the ultrasound probe in the ultrasound observation apparatus shown in Fig. 1;

Fig. 12 is an enlarged perspective view of a principal portion that illustrates, in an enlarged manner, a proximal end portion of the insertion assisting instrument in the ultrasound observation apparatus shown in Fig. 1;

Fig. 13 is an enlarged perspective view of a principal portion that illustrates, in an enlarged manner, the vicinity of the distal end portion of the ultrasound probe in the ultrasound observation apparatus shown in Fig. 1 (approximately the same as Fig. 5);

Fig. 14 is a sectional view at a first portion that is denoted by reference symbol [14] in Fig. 13;

Fig. 15 is a sectional view at a third portion that is denoted by reference symbol [15] in Fig. 13;

Fig. 16 is a sectional view at a fourth portion that is denoted by reference symbol [16] in Fig. 13;

Fig. 17 is a sectional view at a second portion that is denoted by reference symbol [17] in Fig. 13;

Fig. 18 is a conceptual diagram that illustrates one example among desirable shapes with respect to a cross-sectional shape of the insertion portion of the ultrasound probe of the ultrasound observation apparatus shown in Fig. 1;

Fig. 19 is a conceptual diagram that illustrates another example of the cross-sectional shape of the insertion portion of the ultrasound probe of the ultrasound observation apparatus shown in Fig. 1;

Fig. 20 is a conceptual diagram that illustrates a different example of the cross-sectional shape of the insertion portion of the ultrasound probe of the ultrasound observation apparatus shown in Fig. 1;

Fig. 21 is an external schematic view that illustrates an example of a configuration of a telescope that, after withdrawing an insertion assisting instrument, can be inserted in place thereof in the ultrasound probe in the ultrasound observation apparatus shown in Fig. 1;

Fig. 22 is a conceptual diagram that illustrates the structure of a fixing mechanism for an insertion assisting instrument or the like in the ultrasound probe of the ultrasound observation apparatus shown in Fig. 1;

Fig. 23 is an enlarged longitudinal sectional view of a principal portion along a longitudinal direction of an ultrasound observation apparatus according to a second embodiment of the present invention, that is a sectional view illustrating the internal configuration in the vicinity of a distal end portion of the ultrasound observation apparatus;

Fig. 24 is an enlarged longitudinal sectional view of a principal portion along a longitudinal direction of an ultrasound observation apparatus according to a third embodiment of the present invention, that is a sectional view illustrating the internal configuration in the vicinity of a distal end portion of the ultrasound observation apparatus;

Fig. 25 is an enlarged perspective view of a principal portion that illustrates a modification of the vicinity of the distal end portion of the ultrasound probe in the ultrasound observation apparatus according to the first embodiment of the present invention; and

Fig. 26 is a sectional view at a third portion that is denoted by reference symbol [26] in Fig. 25.

Best Mode for Carrying Out the Invention

The present invention is described hereunder by way of embodiments that are illustrated in the accompanying drawings. Note that in some cases the respective components in the drawings used in the following description may be displayed in a different contraction scale so as to be shown in a size that is recognizable in the drawings. Accordingly, the present invention is not limited only to the quantity of components, the shapes of components, the ratios between the sizes of components, and the relative positional relationship between the respective components described in the drawings.

[First Embodiment]

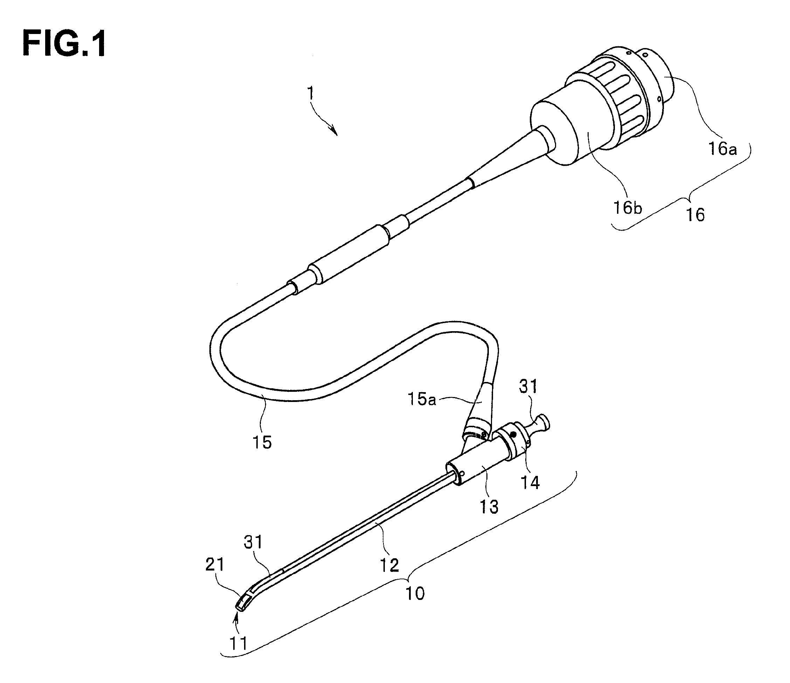

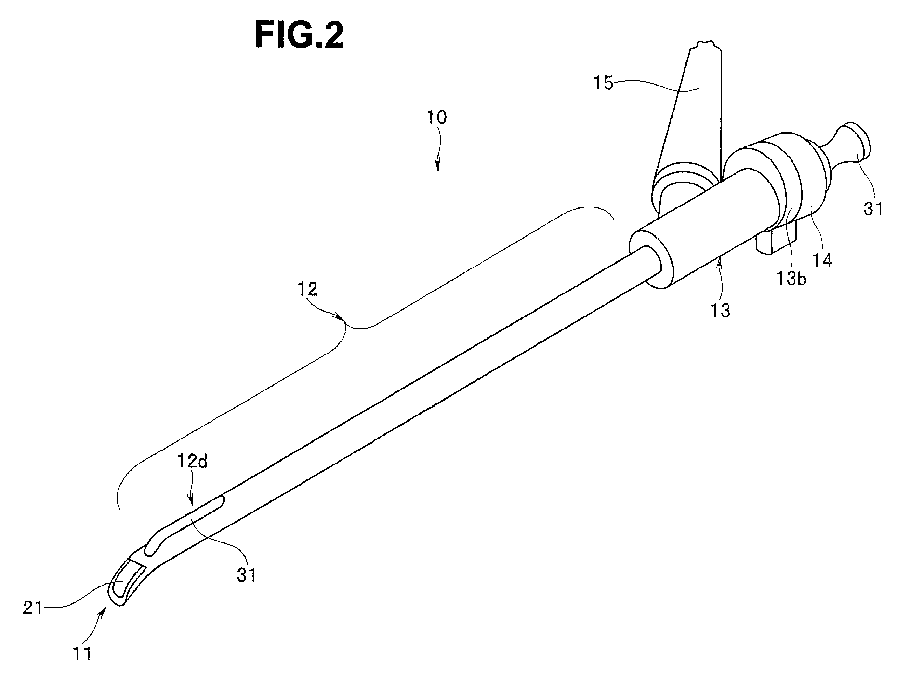

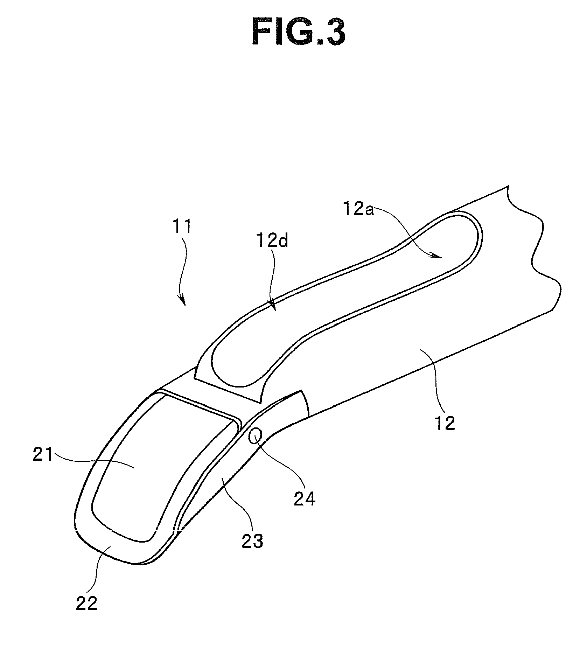

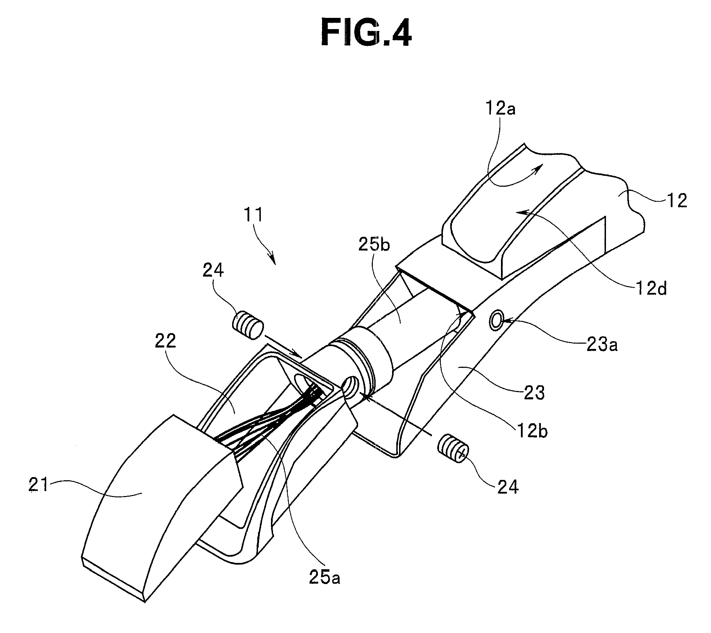

Fig. 1 to Fig. 22 are views that illustrate an ultrasound observation apparatus according to a first embodiment of the present invention. Among these, Fig. 1 is a perspective view that illustrates the overall configuration of the ultrasound observation apparatus of the present embodiment. Fig. 2 is an enlarged perspective view of a principal portion that illustrates, in an enlarged manner, an ultrasound probe in a state in which an insertion assisting instrument is mounted thereto in the ultrasound observation apparatus of the present embodiment. Fig. 3 is an enlarged perspective view of a principal portion that illustrates, in an enlarged manner, the vicinity of a distal end portion of the ultrasound probe in the ultrasound observation apparatus of the present embodiment. Fig. 4 is an exposed perspective view of a principal portion that illustrates, in an exposed manner, components of the distal end portion of the ultrasound probe in the ultrasound observation apparatus of the present embodiment. Note that in Fig. 3 and Fig. 4 a state is illustrated in which the insertion assisting instrument is not mounted to the ultrasound probe.

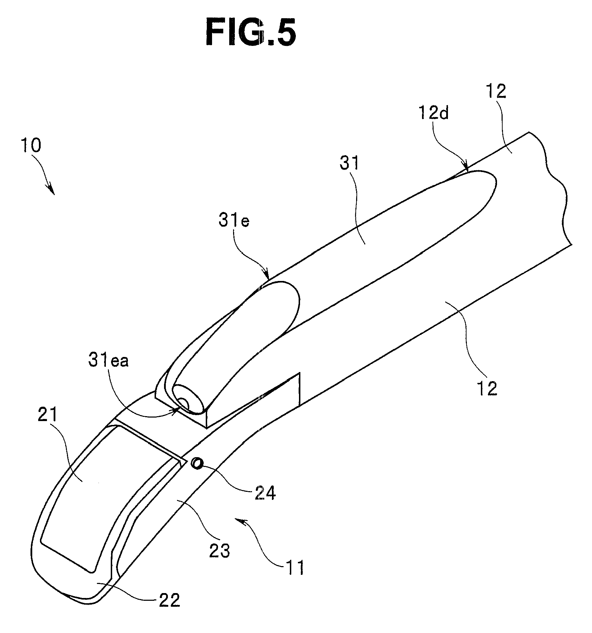

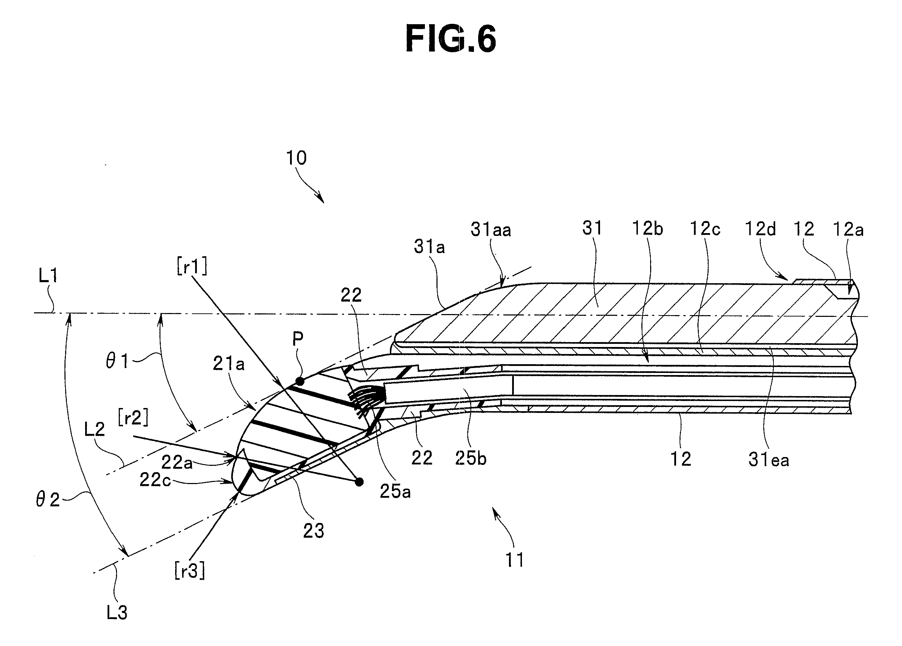

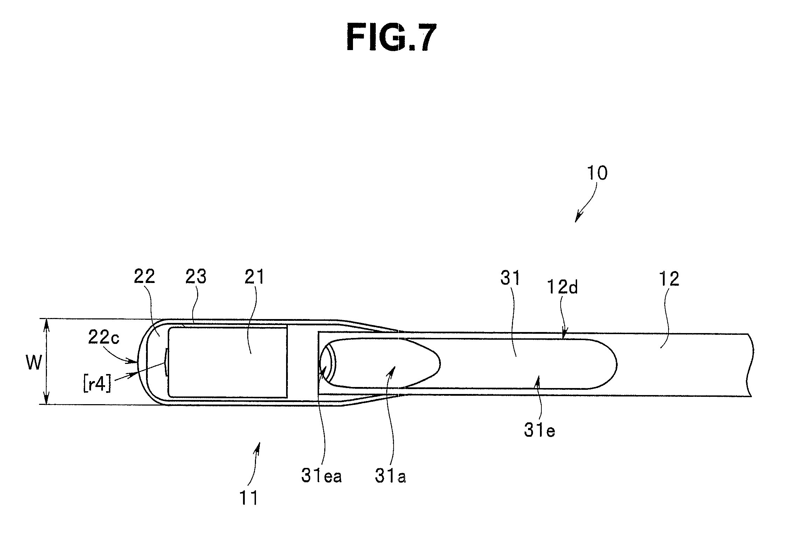

Fig. 5 is an enlarged perspective view of a principal portion that illustrates, in an enlarged manner, the vicinity of the distal end portion of the ultrasound probe in the ultrasound observation apparatus of the present embodiment. Fig. 6 is an enlarged longitudinal sectional view of a principal portion along a longitudinal direction of the ultrasound probe in the ultrasound observation apparatus of the present embodiment, that is a sectional view that illustrates the internal configuration in the vicinity of the distal end portion of the ultrasound observation probe. Fig. 7 is a plan view when viewing the vicinity of the distal end portion of the ultrasound probe in the ultrasound observation apparatus of the present embodiment from a top surface side. Note that in Fig. 6 and Fig. 7 a state is illustrated in which the insertion assisting instrument is mounted to the ultrasound probe.

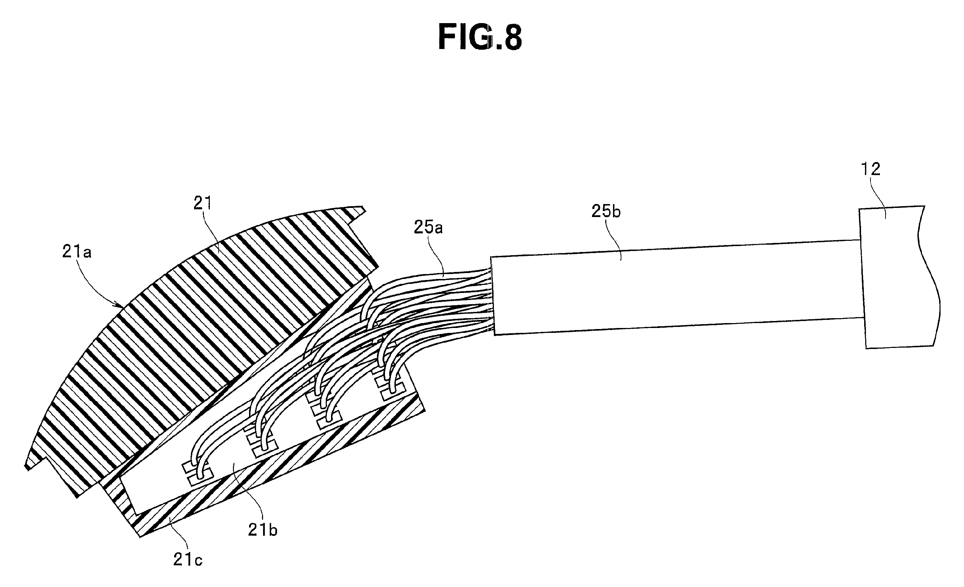

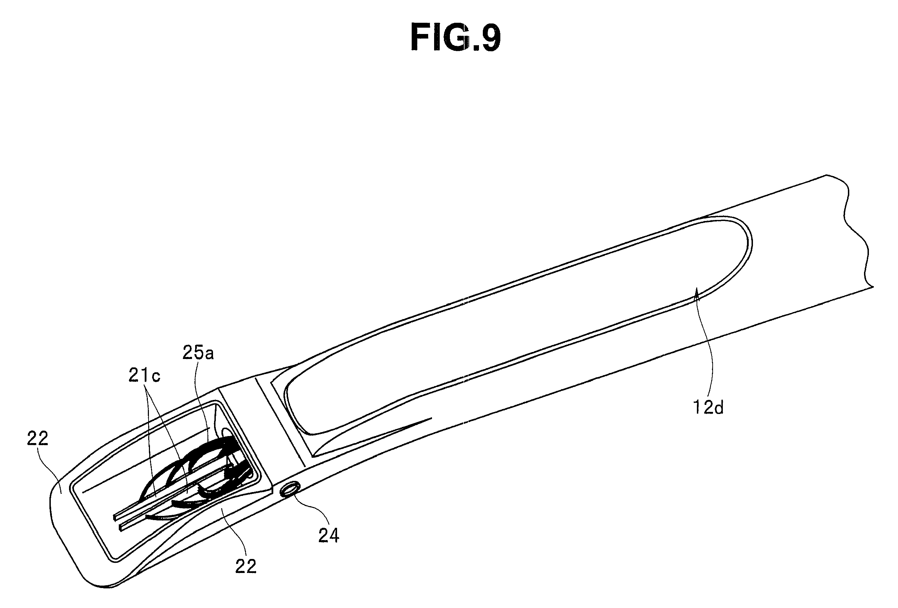

Fig. 8 is an enlarged view of a principal portion that shows constituent members taken out from an ultrasound transducer unit arranged at the distal end portion of the ultrasound probe in the ultrasound observation apparatus of the present embodiment. Fig. 9 is an enlarged perspective view of a principal portion that illustrates components, excluding the ultrasound transducer, of the distal end portion of the ultrasound probe in the ultrasound observation apparatus of the present embodiment.

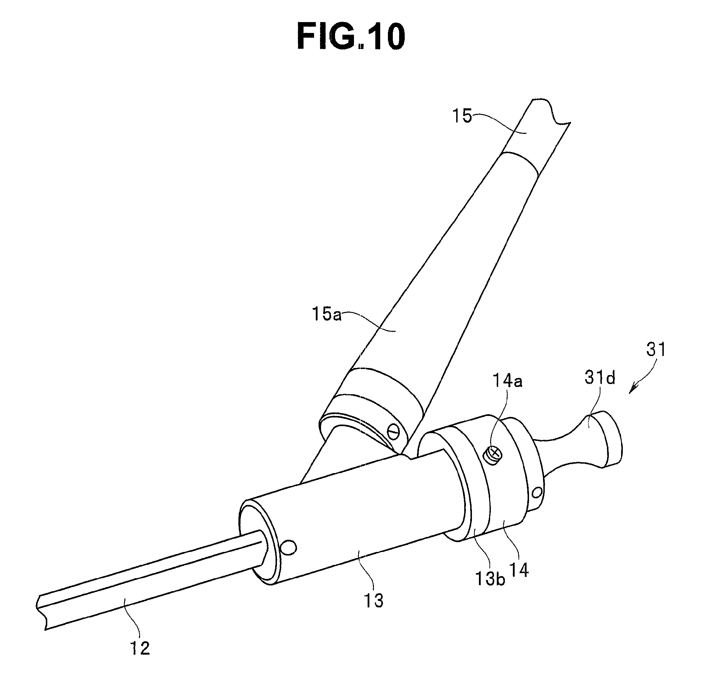

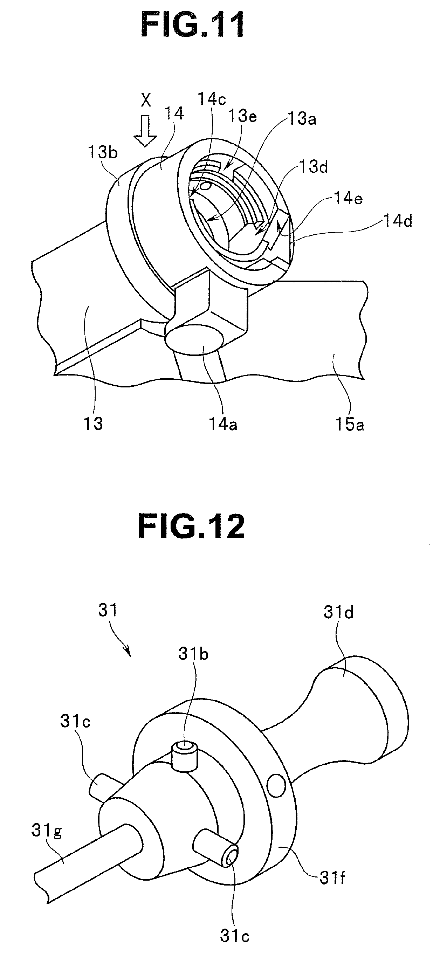

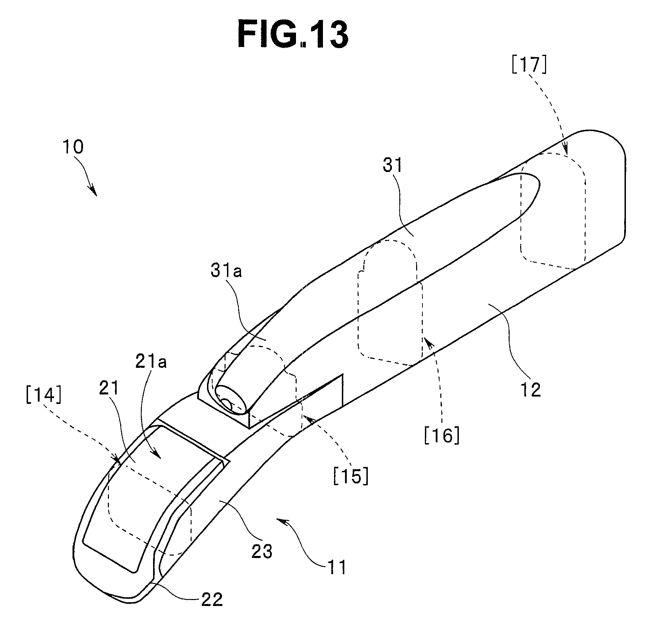

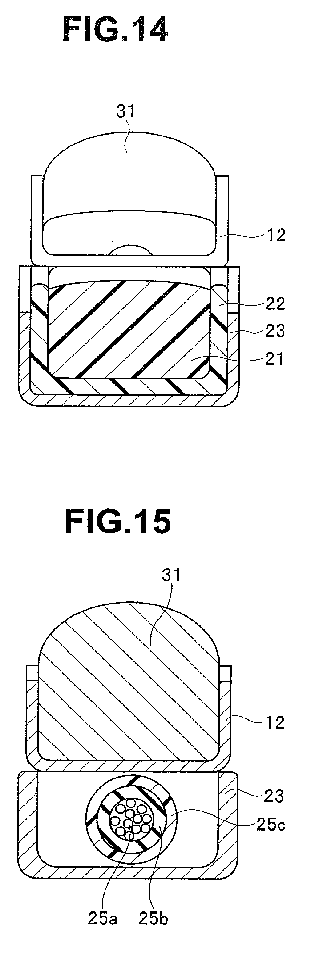

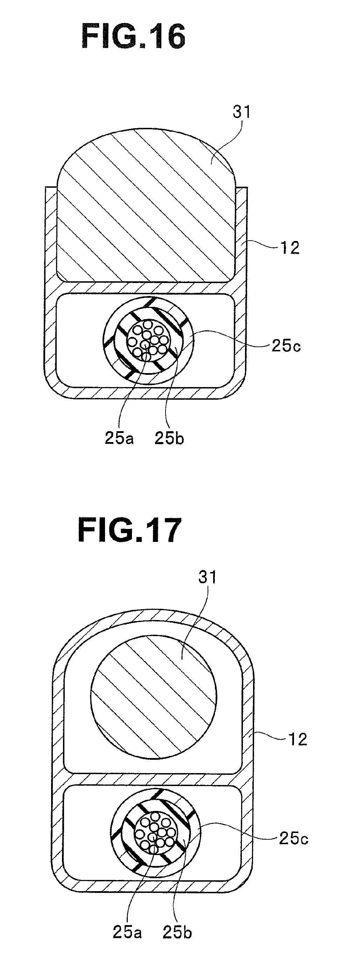

Fig. 10 is an enlarged perspective view of a principal portion that illustrates, in an enlarged manner, a vicinity of the proximal end portion of the ultrasound probe in a state in which the insertion assisting instrument is mounted thereto in the ultrasound observation apparatus of the present embodiment. Fig. 11 is an enlarged perspective view of a principal portion that illustrates, in a further enlarged manner, the vicinity of the proximal end portion of the ultrasound probe in the ultrasound observation apparatus of the present embodiment. Note that in Fig. 11 a state is illustrated in which the insertion assisting instrument is not mounted to the ultrasound probe. Fig. 12 is an enlarged perspective view of a principal portion that illustrates, in an enlarged manner, a proximal end portion of the insertion assisting instrument in the ultrasound observation apparatus of the present embodiment. Fig. 13 is an enlarged perspective view of a principal portion that illustrates, in an enlarged manner, the vicinity of the distal end portion of the ultrasound probe in the ultrasound observation apparatus of the present embodiment (approximately the same as Fig. 5). Fig. 14 is a sectional view at a first portion that is denoted by reference symbol [14] in Fig. 13. Fig. 15 is a sectional view at a third portion that is denoted by reference symbol [15] in Fig. 13. Fig. 16 is a sectional view at a fourth portion that is denoted by reference symbol [16] in Fig. 13. Fig. 17 is a sectional view at a second portion that is denoted by reference symbol [17] in Fig. 13.

First, an outline of the overall configuration of the ultrasound observation apparatus of the present embodiment will be described using Fig. 1. As shown in Fig. 1, an ultrasound observation apparatus 1 of the present embodiment is mainly constituted by: an ultrasound probe 10 that includes a distal end portion 11, an insertion portion 12, a grasping portion 13, and a fixing slider 14; an insertion assisting instrument 31, also referred to as a "obturator" or the like, that is insertedly disposed in the ultrasound probe 10; an observation apparatus connecting tube 15 that is extended from the grasping portion 13 of the ultrasound probe 10; and the respective components of an observation apparatus connecting connector 16 provided in a linked manner to a distal end of the observation apparatus connecting tube 15. According to this configuration, the ultrasound probe 10 is connected to an unshown ultrasound observation apparatus unit through the observation apparatus connecting tube 15 and the observation apparatus connecting connector 16. The ultrasound observation apparatus unit is configured to enable acquisition and observation of an ultrasound image of a desired subject by controlling an ultrasound observation unit (described in detail later) that is arranged at a distal end of the ultrasound probe 10 to perform transmission/reception of ultrasound.

As described later, an insertion channel 12a (see Fig. 3 and the like) into which the insertion assisting instrument 31, a telescope (one kind of optical endoscope), an endoscope or the like is insertedly disposed is formed in the ultrasound probe 10. A configuration is adopted such that, at a time of an operation to insert the ultrasound probe 10 into a body cavity of a subject, by performing the operation is a state in which the insertion assisting instrument 31 (obturator) is inserted into the insertion channel 12a, the insertion operation can be smoothly performed when inserting the ultrasound probe 10 into the body cavity of the subject. Further, at a time of an operation to observe a subject using the ultrasound probe 10, observation of the subject can be performed by insertedly disposing a telescope or an endoscope or the like in the insertion channel 12a.

That is, although the ultrasound probe 10 is a shape which, because of having the insertion channel 12a, is difficult to insert into a body cavity of a subject as it is, operations at the time of an insertion operation can be performed smoothly by insertedly disposing into the insertion channel 12a the insertion assisting instrument 31 that is an assisting instrument that assists an insertion operation and smoothens the insertion process.

In addition, when the insertion assisting instrument 31 (obturator) is withdrawn after inserting the ultrasound probe 10 into the subject, fluids or the like that have accumulated inside the subject can be sampled in one go through the insertion channel 12a. Further, after withdrawing the insertion assisting instrument 31, if a telescope or endoscope or the like is inserted into the insertion channel 12a, observation of the subject inside the body cavity can be performed using visible light or special light or the like. Further, if a treatment instrument such as a puncture needle is inserted into the insertion channel 12a, desired treatment can be performed on the subject inside the body cavity, or tissue of a desired region inside the body cavity can be sampled.

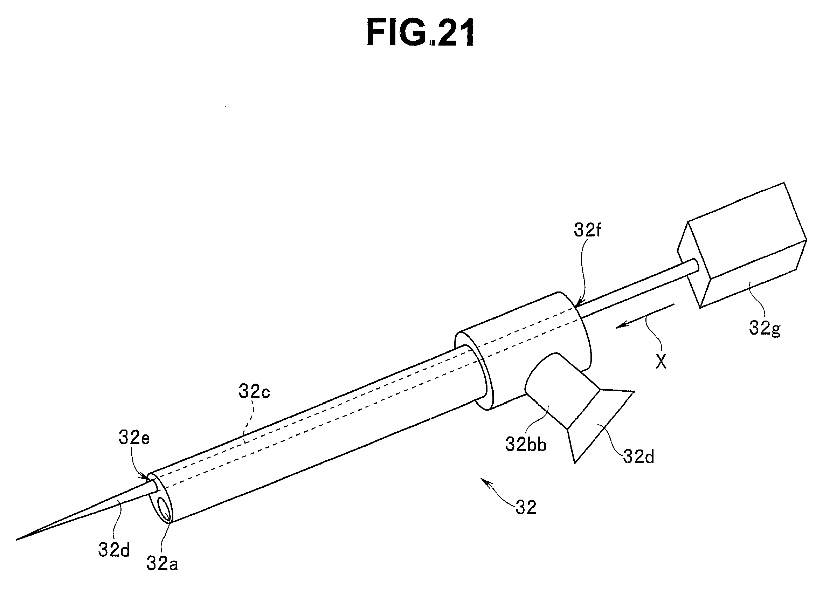

For example, Fig. 21 is an external schematic view that illustrates one example of a form of a telescope that, after withdrawal of the insertion assisting instrument 31, can be inserted in place thereof in the ultrasound probe in the ultrasound observation apparatus of the present embodiment.

A telescope 32 shown in Fig. 21 is formed in a tubular shape that can be inserted through the insertion channel 12a of the insertion portion 12 of the ultrasound probe 10. The telescope 32 has an objective optical system 32a as an optical observation portion at a distal end portion thereof, and an ocular optical system 32b at a proximal end portion. The objective optical system 32a and the ocular optical system 32b have a configuration with which an optical image of a subject can be observed by means of an unshown relay optical system or image guide fiber or the like.

The telescope 32 also includes a treatment instrument channel 32c (second channel). The channel 32c is formed so as to pass through a region from a proximal end side opening 32f to a distal end side opening 32e. The channel 32c is configured so that a treatment instrument such as a puncture needle 32d that is extended from a treatment instrument apparatus (puncture needle system box) 32g can be inserted therethrough in the direction of an arrow X in Fig. 21.

By using the telescope 32 having this form, a biopsy can be performed by means of the puncture needle 32d while simultaneously performing optical observation in addition to ultrasound observation with the ultrasound probe 10.

In this case, in order to reliably carry out operation of the puncture needle 32d without hindrance while performing optical observation, it is desirable to adopt a configuration such that the treatment instrument channel 32c and an ocular barrel 32bb of the ocular optical system 32b are not parallel to each other. In the example shown in Fig. 21, the ocular barrel 32bb of the ocular optical system 32b is, for example, arranged so as to extend obliquely rearward from the proximal end portion.

Note that although an example of a telescope is shown as one kind of optical endoscope using Fig. 21, the form of a telescope or an endoscope that can be applied to the ultrasound observation apparatus 1 of the present embodiment is not limited thereto, and telescopes or endoscopes of other configurations can be widely applied as long as the telescope or endoscope has a tubular form that can be inserted through the insertion channel 12a of the insertion portion 12 of the ultrasound probe 10.

Next, the detailed configurations of the ultrasound probe and the insertion assisting instrument of the ultrasound observation apparatus of the present embodiment will be described using the drawings. Note that the detailed configurations mainly on the distal end side of the ultrasound probe and the insertion assisting instrument are described using Fig. 2 to Fig. 9 and Fig. 13 to Fig. 17. Further, the detailed configurations mainly on the proximal end side of the ultrasound probe and the insertion assisting instrument, particularly a fixing mechanism (fixing slider 14 and the like) for fixing the insertion assisting instrument 31 and the like with respect to the grasping portion 13 are described using Fig. 10 to Fig. 12.

The distal end portion 11 is mainly constituted by an ultrasound observation unit that includes a convex-type ultrasound transducer 21 that is an ultrasound transmitting/receiving portion that has an ultrasound transmitting/receiving face 21a (see Fig. 6) that transmits/receives ultrasound, a resin-made housing 22 that is a holding part (housing) that fixedly holds the ultrasound transducer 21, and a metal-made housing 23 that constitutes one part of the holding part (housing) and is formed so as to cover the outer surface of the resin-made housing 22.

As shown in Fig. 6, Fig. 8, Fig. 9 and the like, a plurality of wires 25a are connected to the ultrasound transducer 21. The plurality of wires 25a, for example, are covered by a flexible coil made of metal and a tube made of resin, and are further bundled using an ultrasound cable bundle 25b made of an electrically insulative material. The external surface side of the ultrasound cable bundle 25b is further covered by a cable lumen 25c (see Fig. 16) to thereby form a single ultrasound cable. As shown in Fig. 8 and Fig. 9, the plurality of wires 25a are respectively connected on an electric board 21b. The electric board 21b is covered by a packing material 21 c. The packing material 21 c is integrally arranged on a bottom face of the ultrasound transducer 21.

Although not illustrated in the drawings, the ultrasound transducer 21 includes, for example, an upper electrode, a piezoelectric element, and a lower electrode and the like in that order from the surface thereof. An acoustic matching layer and a protective film are formed on the surface of the upper electrode. Note that an ultrasound observation unit that is substantially the same as an ultrasound observation unit used in an ultrasound observation apparatus that is already in widespread use is applied as the ultrasound observation unit with this configuration.

The ultrasound observation unit with this configuration generates ultrasound by sending electricity to the electrodes through the wires 25a to drive the piezoelectric element. Further, the piezoelectric element converts received ultrasound to electricity and sends the electricity to the ultrasound observation apparatus (not shown) through the wires 25a to thereby enable formation of an ultrasound image. The wires 25a pass through, as the ultrasound cable bundle 25b, an internal space (12b) of the insertion portion 12 of the ultrasound probe 10, and are connected to the observation apparatus connecting connector 16 through the observation apparatus connecting tube 15 at a rear end of the ultrasound probe 10.

The aforementioned resin-made housing 22 is a case member that holds the ultrasound transducer 21, and is formed by a member made of resin that has electrical insulation properties. An opening is formed at the rear end of the resin-made housing 22. An ultrasound cable that includes the plurality of wires 25a and the like extends from this opening (see Fig. 6). The metal-made housing 23 that is formed by a high-strength member, for example, a metal member made of stainless steel (SUS), titanium (Ti) or the like, is arranged on faces other than the upper face of the external surface of the resin-made housing 22. The metal-made housing 23 is formed in a shape in which the distal end side of the insertion portion 12 is extended.

The resin-made housing 22 is fixedly installed by means of a fixing screw 24 (see Fig. 3 and Fig. 5) in the metal-made housing 23. A male thread is formed in the fixing screw 24. When fixedly installing the metal-made housing 23 at the distal end of the insertion portion 12, the fixing screw 24 screws together with a screw hole 23a of the metal-made housing 23 and serves to provide the aforementioned two components in a fixed condition with respect to each other. Further, to reliably ensure electrical insulation between the resin-made housing 22 that holds the ultrasound transducer 21, and the insertion portion 12 and metal-made housing 23 that are made of metal, the fixing screw 24 is, for example, formed by a resin raw material that has electrical insulation properties.

The insertion portion 12 is, for example, formed in a rigid cylindrical shape using a metal member such as stainless (SUS), titanium (Ti) or the like. The ultrasound observation unit that includes the aforementioned holding part such as the resin-made housing 22 and the metal-made housing 23 is fixedly installed at a position that is furthest on the distal end side of the insertion portion 12. The grasping portion 13 is provided in a linked manner to the proximal end side of the insertion portion 12 (see Fig. 1 and Fig. 2).

The insertion portion 12 is formed in a hollow, substantially linear cylindrical shape. As shown in Fig. 6 and the like, a partition wall 12c that divides the internal space into an upper-side space and a lower-side space along the longitudinal direction is formed at approximately the center of the inside of the insertion portion 12. Thus, the internal space of the insertion portion 12 has a configuration in which two chambers extend in the longitudinal direction. Of these, the ultrasound cable is inserted through the internal space (denoted by reference symbol 12b) on the lower side of the insertion portion 12 from the distal end portion 11 to the grasping portion 13 on the proximal end side. Therefore, in the following description, the aforementioned internal space that is denoted by reference symbol 12b is referred to as "cable insertion path 12b". Note that the ultrasound cable is inserted through the inside of the observation apparatus connecting tube 15 that extends from the side of the grasping portion 13, and reaches the unshown ultrasound observation apparatus and is electrically connected thereto.

On the other hand, the insertion assisting instrument 31 or an unshown telescope or endoscope or the like is insertedly disposed in the internal space (denoted by reference symbol 12a) on the upper side of the insertion portion 12. Therefore, in the following description, the aforementioned upper-side internal space that is denoted by reference symbol 12a is referred to as "insertion channel 12a".

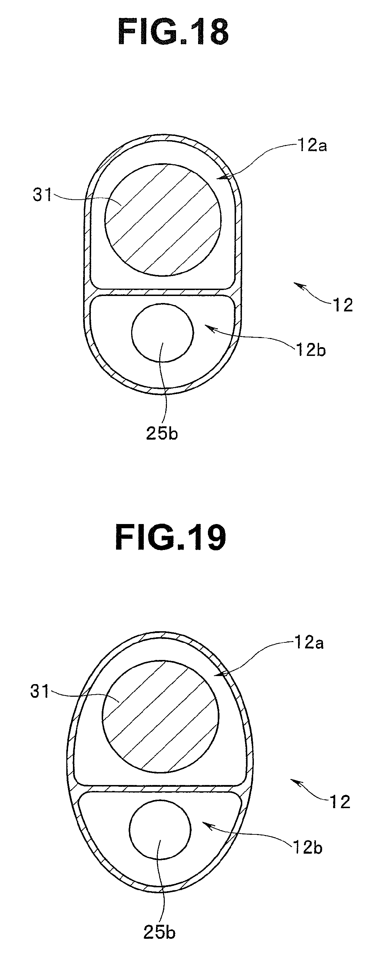

The cross-sectional shape of the insertion portion 12 and the positional relationship between the insertion channel 12a and the cable insertion path 12b will now be described. Fig. 18 to Fig. 20 are views that conceptually illustrate three examples of a desirable shape with respect to the cross-sectional shape of the insertion portion of the ultrasound probe of the ultrasound observation apparatus of the present embodiment. At the same time, Fig. 18 to Fig. 20 also illustrate the positional relationship between the insertion channel 12a and the cable insertion path 12b at the cross section of the insertion portion 12.

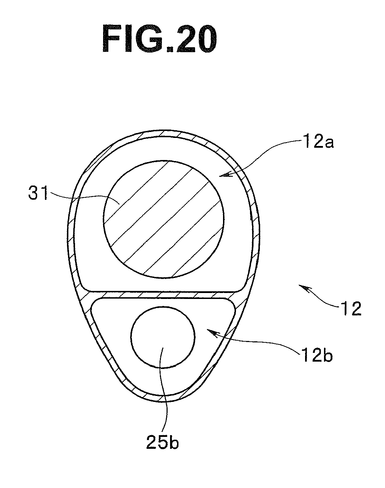

As described above, the insertion portion 12 has the partition wall 12c at an approximately central portion inside the hollow, substantially linear cylindrical shape, to thereby form the cable insertion path 12b that is the lower-side space and the insertion channel 12a that is the upper-side space. Accordingly, the cross-sectional shape of the insertion portion 12 is long in the vertical direction. When a cross-sectional shape having this kind of shape and that does not hinder insertability is considered, it is desirable that the cross-sectional shape of the insertion portion 12 of the ultrasound probe 10 be formed in, for example, an oblong shape as shown in Fig. 18, an elliptic shape as shown in Fig. 19, or an egg shape as shown in Fig. 20. Further, with respect to the insertion channel 12a and the cable insertion path 12b, since the former is configured to be wider than the latter, it is desirable that the positional relationship between the insertion channel 12a and the cable insertion path 12b when shapes as in the above described three forms (Fig. 18 to Fig. 20) are adopted is such that, as shown in the drawings, the insertion channel 12a is arranged in a region in which the cross-sectional area is larger.

In addition, in the vicinity of the distal end side of the insertion portion 12, a channel opening portion 12d that communicates with the insertion channel 12a is formed in a form in which the distal end side and a part of the upper side are notched. One part of the distal end side of the insertion assisting instrument 31 is disposed so as to be exposed in the channel opening portion 12d. Therefore, the distal end shape of the insertion portion 12 is formed so as to have the shape of an inclined face that inclines relative to the longitudinal direction of the insertion portion 12, in conformity with a distal-end inclined face 31a (described in detail later) of the insertion assisting instrument 31.

Note that although the width dimension in the horizontal direction of the ultrasound observation unit that is fixedly installed on the distal end side of the insertion portion 12 is formed so as to have a wide width to ensure the performance of the ultrasound transducer 21, by suppressing the height dimension in the vertical direction to a low height, the perimeter thereof is formed so as to be less than the perimeter of the insertion portion 12. Note that the term "perimeter" as used in this case refers to the outer circumferential length at a cross section in a direction that is perpendicular to the longitudinal direction of the insertion portion 12 (insertion direction) in a state in which the insertion assisting instrument 31 is insertedly disposed in the insertion portion 12.

That is, at a distal end portion of the ultrasound observation unit, that is, at least at a predetermined region (for example, a region as far as a portion on the proximal end side of the channel opening portion 12d) towards the distal end of the insertion portion 12 from the distal end portion 11, the perimeter is formed so as to gradually increase from the distal end side towards the proximal end side. Further, in this case, a configuration may also be adopted in which the perimeter of the same region is uniformly formed.

More specifically, for example, a form as shown in Fig. 13 to Fig. 17 may be adopted. In this case, first, a portion denoted by reference symbol [14] in Fig. 13 (see the sectional view in Fig. 14) is taken as a first portion that is furthest towards the distal end. The first portion is a portion in the vicinity of an approximately central part in the longitudinal direction of the distal end portion 11 (holding part). In addition, a portion denoted by reference symbol [17] in Fig. 13 (see the sectional view in Fig. 17) is taken as a second portion that is towards the proximal end. This second portion is a portion in the vicinity of the proximal end side of the channel opening portion 12d of the insertion portion 12.

Further, a portion in the vicinity of an approximately central part of the distal-end inclined face 31a of the insertion assisting instrument 31 that is a portion denoted by reference symbol [15] in Fig. 13 (see the sectional view in Fig. 15) that is positioned between the first portion and the second portion is taken as a third portion.

Similarly, a portion in the vicinity of an approximately central part of the channel opening portion 12d of the insertion portion 12 that is a portion denoted by reference symbol [16] in Fig. 13 (see the sectional view in Fig. 16) that is positioned between the first portion and the second portion is taken as a fourth portion.

In this case, the distal end portion 11 of the ultrasound observation apparatus 1 of the present embodiment is formed so that the perimeter of the second portion that is towards the proximal end is greater than the perimeter of the first portion that is furthest towards the distal end.

In addition, the ultrasound observation apparatus 1 of the present embodiment is formed so that a cross-sectional perimeter thereof changes so as to increase in a stepwise manner in at least four steps, or alternatively in a gradual manner, from the first portion on the distal end side towards the proximal end side in the order of the third portion, the fourth portion, and the second portion.

The grasping portion 13 is a grip portion that the user grasps with the fingers or the like when using the ultrasound observation apparatus 1. The grasping portion 13 is formed in a hollow, approximately cylindrical shape using a metal member. The proximal end of the insertion portion 12 is provided in a linked manner to the distal end of the grasping portion 13. Thus, the insertion channel 12a and cable insertion path 12b of the insertion portion 12 communicate with the internal space of the grasping portion 13. A grasping portion opening 13a that communicates with the aforementioned internal space is formed in a rear end face of the grasping portion 13. The grasping portion opening 13a communicates with the insertion channel 12a.

According to this configuration, after the insertion assisting instrument 31, telescope, endoscope or the like that has been inserted from the grasping portion opening 13a passes through the internal space of the grasping portion 13, the insertion assisting instrument 31, telescope, endoscope or the like is insertedly disposed in the insertion channel 12a on the upper side of the insertion portion 12. That is, the grasping portion opening 13a serves as an insertion port for inserting a tubular constituent member such as the insertion assisting instrument 31, a telescope or an endoscope into the insertion channel 12a.

Further, at the side face of the grasping portion 13, a bend preventing portion 15a that is an observation apparatus connector connection portion branches in an obliquely rearward direction from an axis line of the grasping portion 13. The observation apparatus connecting tube 15 that extends from the grasping portion 13 is inserted through the inside of the bend preventing portion 15a. The bend preventing portion 15a functions as tube protection means that is provided in order to prevent the observation apparatus connecting tube 15 that is extended from the grasping portion 13 from bending at the extension portion when using the ultrasound observation apparatus 1. The bend preventing portion 15a is also configured to function as a second grasping portion that a user can grasp when using the ultrasound observation apparatus 1. For this purpose, the bend preventing portion 15a is formed, for example, by a resin member that has a combination of a moderate hardness and flexibility, and is formed integrally with the grasping portion 13.

Thus, in the ultrasound observation apparatus 1 of the present embodiment, since the bend preventing portion 15a that is the observation apparatus connector connection portion is configured so as to be usable as a second grasping portion, for example, while grasping the second grasping portion (bend preventing portion 15a) with one hand to thereby firmly and securely grasp the ultrasound probe 10, a user can, with the other hand, reliably operate a treatment instrument or the like such as a puncture needle in an endoscope or the like that has been inserted through the insertion channel 12a. Accordingly, operation of the ultrasound probe 10 and operation of a treatment instrument or the like such as a puncture needle can be reliably performed by a single person.

Further, since the user can firmly grasp the ultrasound probe 10 by grasping the second grasping portion, a situation does not occur in which the user drops the ultrasound probe 10 at the time of an ultrasound observation operation.

Furthermore, since the bend preventing portion 15a as the second grasping portion is formed so as to extend obliquely rearward from a side portion of the grasping portion 13, the shape is designed so as not to be a hindrance to the user at a time of use.

As described above, the ultrasound cable that is inserted through the insertion portion 12 from the distal end portion 11 is inserted through the inside of the observation apparatus connecting tube 15 via the grasping portion 13. The observation apparatus connecting connector 16 is arranged at the distal end of the observation apparatus connecting tube 15, and the ultrasound cable is connected to the unshown ultrasound observation apparatus through the observation apparatus connecting connector 16.

A fixing mechanism that includes the fixing slider 14 and the like for fixing the insertion assisting instrument 31, a telescope, an endoscope or the like that is insertedly disposed in the insertion channel 12a is configured in the vicinity of a rear end portion of the grasping portion 13. The detailed configuration of this fixing mechanism is described later (see Fig. 11 and Fig. 12).

The insertion assisting instrument 31 is formed in a rigid rod shape or cylindrical shape that is made from a metal member or the like, and is a member that is insertedly disposed so as to be freely insertable/removable from the grasping portion opening 13a with respect to the insertion portion 12. That is, when the insertion assisting instrument 31 is insertedly disposed inside the insertion portion 12 to form an integral configuration with the insertion portion 12, the insertion assisting instrument 31 serves as a guide member that guides so that the insertion portion 12 can be smoothly inserted from the distal end portion 11 into, for example, a body cavity of a narrow and blocked form such as the urethra.

The insertion assisting instrument 31 has the distal-end inclined face 31a at a portion that is furthest on the distal end side thereof, and is formed by: a distal-end shape portion 31e that is formed in conformity with the internal shape of the insertion portion 12 and is insertedly disposed at a position corresponding to the channel opening portion 12d of the insertion portion 12; a proximal end grasping portion 31d that is formed at a portion that is furthest on a proximal end side thereof; a flange portion 31f that is formed in the vicinity of the proximal end grasping portion 31d; a protrusion for rotational direction positioning 31 b and two engagement protrusions for fixing 31 c that are provided in a protruding manner towards the outer circumference at portions that are towards the distal end of the flange portion 31f; and an intermediate rod-shaped portion 3 1 g that connects the distal-end shape portion 31e and the proximal end grasping portion 31d.

A groove portion 31ea is formed in the distal-end shape portion 31e of the insertion assisting instrument 31 (see Fig. 5, Fig. 6 and the like). The groove portion 31ea extends in the longitudinal direction in a face on a side that faces the partition wall 12c when the insertion assisting instrument 31 is inserted inside the insertion channel 12a of the insertion portion 12. The groove portion 31ea constitutes one part of a lubricant supply path that allows a lubricant such as, for example, Xylocaine jelly that is supplied via an unshown lubricant supply mechanism to pass through to the distal end side. The lubricant is used to aid insertion of the insertion portion 12 into the urethra or the like.

Although a detailed illustration of the lubricant supply mechanism is omitted from the drawings, an outline of the configuration thereof is described hereunder.

That is, for example, the lubricant supply mechanism includes a cock member that forms a portion that connects a lubricant supply tube to the grasping portion 13, and also serves to open/close the lubricant supply tube. The lubricant supply tube is connected to an unshown lubricant supply apparatus or the like. With this configuration, lubricant that is supplied from the lubricant supply apparatus or the like passes through the lubricant supply tube and, by placing the cock member in an open state, is injected into the grasping portion 13. The lubricant that has been injected into the internal space of the grasping portion 13 is led to the distal end side via the inside of the insertion channel 12a of the insertion portion 12, and is led to the outside from the most distal end portion of the distal-end shape portion 3 1 e of the insertion assisting instrument 31 through the groove portion 31ea.

Note that although Fig. 1, Fig. 2, Figs. 5 to 7, and Fig. 13 illustrate a state in which the insertion assisting instrument 31 has been insertedly disposed in the insertion portion 12, in Fig. 3, Fig. 4, and Fig. 9 an illustration of the insertion assisting instrument 31 is omitted to avoid complicating the drawings.

Next, the detailed configuration of the fixing mechanism that is configured in the vicinity of the rear end portion of the grasping portion 13 is described using mainly Fig. 11 and Fig. 12.

As shown in Fig. 11, the fixing mechanism includes a rear end flange 13b formed in the vicinity of the rear end portion of the grasping portion 13, a cylindrical portion 13c that protrudes further towards the rear end side from the rear end flange 13b, and the fixing slider 14 that fits with the cylindrical portion 13c.

Two engagement grooves for fixing 13d and an engagement groove for rotational direction positioning 13e are formed in the cylindrical portion 13c. The two engagement grooves for fixing 13d and the engagement groove for rotational direction positioning 13e are each a groove portion that extends in the longitudinal direction of the grasping portion 13 (insertion direction of the insertion assisting instrument 31).

The two engagement grooves for fixing 13d are formed at positions that face each other with an interval of a 180-degree angle therebetween in the circumferential direction of the cylindrical portion 13c. The engagement groove for rotational direction positioning 13e is formed at a position that is separated by an angle of approximately 90 degrees in the circumferential direction from each of the two engagement grooves for fixing 13d.

The engagement groove for rotational direction positioning 13e is a groove portion with which the protrusion for rotational direction positioning 31b on the insertion assisting instrument 31 side engages when the insertion assisting instrument 31 is insertedly disposed in the insertion portion 12 through the grasping portion 13.

The two engagement grooves for fixing 13d are groove portions with which the two engagement protrusions for fixing 31 c on the insertion assisting instrument 31 side engage, respectively, when the insertion assisting instrument 31 is insertedly disposed in the insertion portion 12 through the grasping portion 13.

The fixing slider 14 is formed by a ring-shaped metal member, and is arranged on an outer circumferential side of the cylindrical portion 13c. In this case, the fixing slider 14 is mounted in a state in which the fixing slider 14 is urged in the direction of an arrow X in Fig. 11 by an unshown urging member so as to be capable of sliding in a direction (same X direction) that is perpendicular to the longitudinal direction of the grasping portion 13. For this purpose, the inner diameter of the fixing slider 14 is formed so as to be larger than the outer shape of the cylindrical portion 13c. Further, the external diameter of the fixing slider 14 is formed to be approximately the same diameter as the external diameter of the aforementioned flange 13b. An operation knob 14a is provided in an outwardly protruding condition on the outer circumferential face of the fixing slider 14. The protruding direction of the operation knob 14a is arranged to be the same direction as an urging direction of the fixing slider 14. That is, the configuration is such that the fixing slider 14 can be slidingly moved by operating the operation knob 14a by pressing the operation knob 14a in the opposite direction against an urging force of the fixing slider 14.

Two guide grooves 14d are formed on an inner circumferential side of the fixing slider 14. The two guide grooves 14d are guiding grooves that serve to lead the two engagement protrusions for fixing 31 c of the insertion assisting instrument 31 to the engagement grooves for fixing 13d when insertedly disposing the insertion assisting instrument 31 in the insertion portion 12 through the grasping portion 13.

For this purpose, the guide grooves 14d each have an inclining portion 14e that has an inclination with respect to the insertion direction when inserting the insertion assisting instrument 31 into the grasping portion 13. Further, the guide grooves 14d are formed so as to communicate with the engagement grooves for fixing 13d when the fixing slider 14 is moved in the opposite direction to the arrow X against the urging force.

According to this configuration, when the insertion assisting instrument 31 is inserted into the insertion portion 12 through the grasping portion 13, the protrusion for rotational direction positioning 31 b engages with the engagement groove for rotational direction positioning 13e to thereby regulate rotation of the insertion assisting instrument 31.

Simultaneously therewith, the engagement protrusions for fixing 31c enter the guide grooves 14d and contact against the respective inclining portions 14e. If the insertion assisting instrument 31 is pushed further in the insertion direction from this state, the fixing slider 14 slidingly moves in the opposite direction to the arrow X against the urging force. As a result the guide grooves 14d and the engagement grooves for fixing 13d enter a communicating state, and hence the engagement protrusions for fixing 31c are led to the engagement grooves for fixing 13d. Subsequently, when the engagement protrusions for fixing 31c pass over the inclining portions 14e of the guide grooves 14d and enter into the engagement grooves for fixing 13d, the fixing slider 14 is slidingly moved in the arrow X direction by the urging force and returns to its original position. As a result, a state is entered in which opening portions of the engagement grooves for fixing 13d are blocked off by the fixing slider 14. That is, the communicating state between the engagement grooves for fixing 13d and the guide grooves 14d is blocked. Consequently, the insertion assisting instrument 31 is fixedly held in a non-rotating state that is also a state in which withdrawal thereof is not possible.

On the other hand, in this state, if the operation knob 14a is pressed in the opposite direction to the arrow X against the urging force of the fixing slider 14 to cause the fixing slider 14 to slidingly move, the engagement grooves for fixing 13d and the guide grooves 14d enter a communicating state. Accordingly, by grasping the proximal end grasping portion 31d of the insertion assisting instrument 31 and moving the insertion assisting instrument 31 in the withdrawal direction while maintaining this state, the insertion assisting instrument 31 can be easily sampled.

The fixing mechanism for fixing the insertion assisting instrument 31, a telescope, an endoscope or the like to one part of the grasping portion 13 will now be described in detail using another drawing.

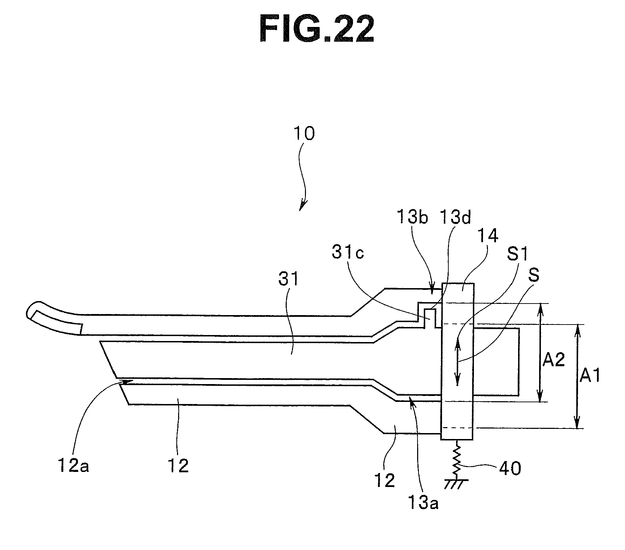

Fig. 22 is a view for describing the structure of the above described fixing mechanism, which conceptually illustrates the internal structure of the ultrasound probe 10 of the ultrasound observation apparatus 1 of the present embodiment. The reference symbols shown in Fig. 22 correspond to the respective constituent members in the foregoing description.

In Fig. 22, the insertion assisting instrument 31 is insertedly disposed in the insertion channel 12a of the ultrasound probe 10 and is in a fixed state.

The grasping portion 13 is provided at the proximal end portion of the ultrasound probe 10. The fixing slider 14 that is capable of slidingly moving in an arrow S direction (direction perpendicular to the axial direction of the ultrasound probe 10) is arranged on the proximal end side of the grasping portion 13. The fixing slider 14 is constantly urged by an urging member 40 in the direction perpendicular to the axial direction of the ultrasound probe 10. Note that although in Fig. 22 the urging member 40 is depicted as being on the outside of the ultrasound probe 10 to simplify the drawing, in fact the urging member 40 is arranged inside the grasping portion 13 of the ultrasound probe 10. Further, the state shown in Fig. 22 is a state in which the urging member 40 is urging the fixing slider 14. In this state, positioning of the fixing slider 14 with respect to the grasping portion 13 is performed by the fixing slider 14 contacting against an unshown fixing portion while receiving the urging force of the urging member 40, or the like.

Further, according to the state shown in Fig. 22, in a state in which the insertion assisting instrument 31 has been insertedly disposed in the insertion channel 12a, the engagement protrusions for fixing 31 c of the insertion assisting instrument 31 are fitted in the engagement grooves for fixing 13d formed in a concave shape of the flange 13b that is one part of the grasping portion 13. In this state, the fixing slider 14 blocks an opening to the rearward side of the engagement grooves for fixing 13d so that the engagement protrusions for fixing 31c do not disengage from the engagement grooves for fixing 13d. At this time, the opening of the fixing slider 14 is an area denoted by reference symbol A1 in Fig. 22.

If the fixing slider 14 is slidingly moved in the direction of an arrow S1 against the urging force of the urging member 40 from the state shown in Fig. 22, the opening of the fixing slider 14 moves to the area denoted by reference symbol A2 in Fig. 22. When this state is entered, since the opening of the fixing slider 14 moves and opens the opening to the rearward side of the engagement grooves for fixing 13d, it is possible for the engagement protrusions for fixing 31c to move rearward. Hence, when this state is entered, the insertion assisting instrument 31 can be withdrawn in the rearward direction.

The above described fixing mechanism can be configured not only in the insertion assisting instrument 31. A similar fixing mechanism can be configured by forming a similar configuration (constituent portions such as the engagement protrusions for fixing 31 c) in a telescope or an endoscope or the like that is to be inserted through the insertion channel 12a of the same ultrasound probe 10.

As described above, the holding part (22, 23; ultrasound observation unit) is fixedly installed at a portion that is furthest on the distal end side of the insertion portion 12. The holding part (22, 23; ultrasound observation unit) is fixedly installed at a distal end portion of the insertion portion 12 so as to have a shape that curves with a predetermined angle θ2 (see Fig. 6) in a direction (see reference symbol L3 in Fig. 6) that separates in a downward direction from the longitudinal direction (see reference symbol L1 in Fig. 6) of the insertion portion 12.

The reason why the holding part (22, 23; ultrasound observation unit) is formed in a curved shape relative to the longitudinal direction of the insertion portion 12 in this manner is as follows.

That is, it is assumed that the ultrasound observation apparatus 1 of the present embodiment is, for example, an apparatus that is mainly used when performing transurethral biopsy by inserting the ultrasound observation apparatus 1 into the urethra or the like. Generally, when performing transurethral biopsy the insertion portion 12 is inserted in a substantially linear manner from the distal end portion 11 while pushing and expanding the urethra that is a narrow lumen in a closed state. Further, the urethra has a visceral shape that bends at a portion from the bulb of the urethra portion to the vicinity of the urethral sphincter. Therefore, in order to smoothly pass the distal end portion 11 of the insertion portion 12 through the urethral bend portion, it is desirable to make the distal end portion 11a curved shape relative to the insertion portion 12. However, if the distal end portion 11 curves to a large extent, it will affect the insertability of the insertion portion 12 in a substantially linear section as far as the prostate gland after the start of insertion into the urethra. Accordingly, in consideration of these facts, it is necessary to set a curvature angle of the distal end portion 11 with respect to the insertion portion 12. Consequently, it is desirable to make the curvature angle θ2 (see Fig. 6) of the holding part (22, 23; ultrasound observation unit) that is a constituent member of the distal end portion 11 with respect to the insertion portion 12, for example, an angle of approximately 20 to 35 degrees to conform to the bending shape of the above described urethral bend portion.

In the ultrasound observation apparatus 1 of the present embodiment, the distal end portion 11 (holding part (22, 23; ultrasound observation unit)) is fixedly installed at the distal end of the insertion portion 12 so as to have a shape that curves with an angle of approximately 20 to 35 degrees in a direction away from the longitudinal direction (insertion direction) of the insertion portion 12.

The mounting angle of the distal end portion 11 with respect to the insertion portion 12 will now be specifically described.

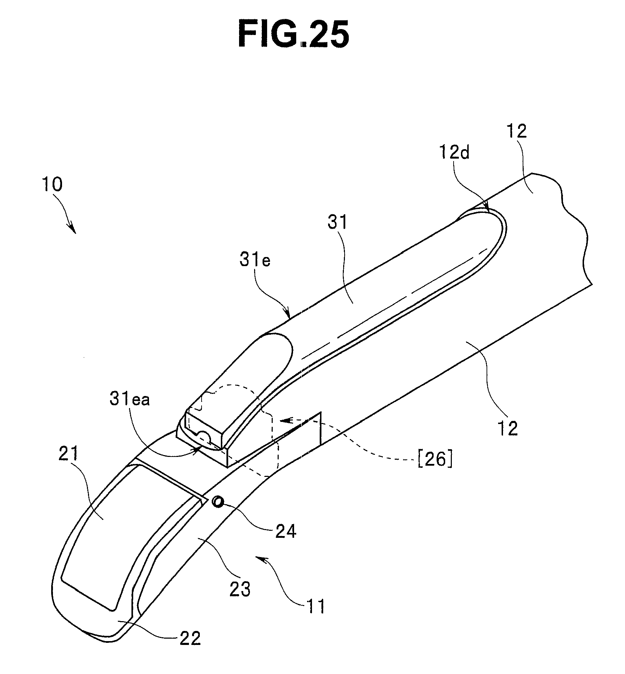

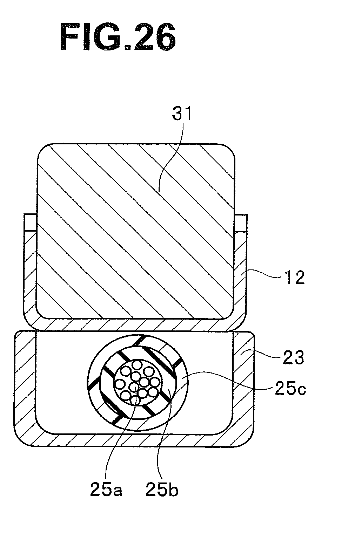

As described above, in the present embodiment, the distal-end inclined face 31a of the insertion assisting instrument 31 is formed by an inclined flat face (or may be formed by an inclined cylindrical face). Fig. 5 and Fig. 15 illustrate examples in which the distal-end inclined face 31a of the insertion assisting instrument 31 is formed with an inclined cylindrical face. Further, Fig. 25 and Fig. 26 illustrate examples in which the distal-end inclined face 3 1 a of the insertion assisting instrument 31 is formed with a flat face.

Fig. 6 illustrates a state in which the insertion assisting instrument 31 has been inserted through the insertion channel 12a of the insertion portion 12. At this time, the channel opening portion 12d of the insertion portion 12 is in a state in which the channel opening portion 12d is blocked by the distal-end shape portion 31e of the insertion assisting instrument 31.

In this case, as shown in Fig. 6, a straight line along the longitudinal direction, that is, a central axis line, of the insertion channel 12a is denoted by reference symbol L1. Further, an extension line of a straight line that contacts the distal-end inclined face 31a (distal end face) of the insertion assisting instrument 31 is denoted by reference symbol L2. In addition, an extension line of a straight line along a surface (back face) of the metal-made housing 23 among the constituent members of the above described holding part is denoted by reference symbol L3. Furthermore, the mounting angle, that is, the curvature angle, of the distal end portion 11 (resin-made housing 22 and metal-made housing 23; ultrasound observation unit) with respect to the insertion portion 12 is denoted by reference symbol θ2.

In the state illustrated in Fig. 6, that is, when the insertion assisting instrument 31 has been insertedly disposed in the insertion portion 12, the distal end portion 11 (holding part) is fixedly installed so that an extension line L2 of a straight line contacting with and running along the distal-end inclined face 31a of the insertion assisting instrument 31 contacts with a single portion on the ultrasound transmitting/receiving face 21 a, that is, a point denoted by reference symbol P in Fig. 6. An angle formed between the central axis line L1 of the insertion channel 12a of the insertion portion 12 and the extension line L2 of the holding part (22, 23) is taken as 81.

In this case, in the ultrasound observation apparatus 1 of the present embodiment, the angle θ1 formed between the extension line L2 of the straight line along the distal-end inclined face 31a of the insertion assisting instrument 31 and the extension line L3 of the straight line along the back face of the metal-made housing 23, and the angle θ2 that is formed between the same extension line L2 and the extension line L3 of a straight line along the surface (back face) of the metal-made housing 23 (holding part) are set to be approximately equal (θ1 ≈ θ2). Note that at this time the extension line L2 of the distal-end inclined face 31a and the extension line L3 of the back face of the metal-made housing 23 are approximately parallel.

On the other hand, as illustrated in the sectional view shown in Fig. 6, the ultrasound transmitting/receiving face 21 a is formed in an arc shape at a cross section in the longitudinal direction of the ultrasound transducer 21. The arc shape of the ultrasound transmitting/receiving face 21a is formed in a continuous shape over the distal end portion of the resin-made housing 22 that holds the ultrasound transducer 21. Here, a portion of the resin-made housing 22 at which the ultrasound transmitting/receiving face 21a and the distal end portion of the resin-made housing 22 are connected is referred to as "distal end continuation portion 22a". The resin-made housing 22 is formed in an arc shape that has a predetermined radius of curvature at a distal end tip portion 22c that is the portion that is furthest on the distal end side.

In this case, reference symbol [r1] shown in Fig. 6 denotes a radius of curvature of the ultrasound transmitting/receiving face 21a at a cross section in the longitudinal direction of the ultrasound transducer 21. Reference symbol [r2] in the same drawing denotes a radius of curvature of the distal end continuation portion 22a at which the ultrasound transmitting/receiving face 21a and the resin-made housing 22 are connected at a cross section in the longitudinal direction of the ultrasound transducer 21. Further, reference symbol [r3] in the same drawing denotes a radius of curvature of the distal end tip portion 22c at a cross section in the longitudinal direction of the resin-made housing 22.

In the ultrasound observation apparatus 1 of the present embodiment, it is desirable to set the relationship between the above described radii of curvature [r1], [r2], and [r3] so that:

$$[r1] \approx [r2] > [r3].$$

More specifically, it is desirable to set the respective radii of curvature so that, for example:

the radius of curvature of the ultrasound transmitting/ receiving face 21a: [r1] = 8 mm approximately; the radius of curvature of the distal end continuation portion 22a: [r2] = 8 mm approximately; and the radius of curvature of the distal end tip portion 22c of the resin-made housing 22 (cross section in Fig. 6): [r3] = 1 mm approximately. By adopting this shape, insertion into the urethra that is a narrow lumen can be performed smoothly.

On the other hand, it is necessary for the ultrasound transducer 21 to have a fixed width dimension due to performance requirements. The term "width dimension" as used herein refers to a dimension denoted by reference character W shown in the plan view in Fig. 7. The distal end tip portion 22c is formed in an arc shape that has a predetermined radius of curvature.

Here, reference symbol [r4] in the plan view in Fig. 7 denotes a radius of curvature in the vicinity of the center in the width direction of the distal end tip portion 22c of the resin-made housing 22. In this case, it is desirable to set the relationship between the respective radii of curvature [r3] and [r4] of the distal end tip portion 22c so that:

$$[r4] > [r3].$$

By adopting this shape, it is possible to inhibit unnecessary lengthening of the most distal end portion of the holding part, that is, the distal end tip portion 22c of the resin-made housing 22, in the longitudinal direction. This can enhance the ability of the distal end tip portion 22c to pass through the urethral bend portion.

Further, it is desirable to also form a cross-sectional shape in the vicinity of an inclination starting point 3 1 aa (see Fig. 6) of the distal-end inclined face 3 1 a of the insertion assisting instrument 31 in an arc shape having a predetermined radius of curvature. Adopting this shape can contribute to enhancing the insertability into the narrow urethra.

Workings when performing transurethral biopsy using the ultrasound observation apparatus 1 of the present embodiment configured as described above will now be described.

First, in a state in which the insertion assisting instrument 31 is insertedly disposed in the insertion portion 12, the user starts insertion of the ultrasound observation apparatus 1 into the urethra by turning over the ultrasound observation apparatus 1 upside down in conformity with the bending shape of the urethra in the vicinity of the urethral sphincter. At this time the user holds the grasping portion 13 and performs an operation to push forward the insertion portion 12. The user may also advance the insertion portion 12 while performing ultrasound observation at the same time as the insertion operation.

When the distal end portion 11 reaches the vicinity of the urethral sphincter after starting insertion, that is, when the distal end portion 11 reaches the urethral bend portion, the user pushes forward the distal end portion 11 in a manner that causes the distal end portion 11 to turn so as to follow the bend portion. After passing through the bend portion and thereafter passing the vicinity of the prostate gland, the distal end portion 11 reaches the urinary bladder. The user can perform these insertion processes while recognizing the state of the relevant processes by means of ultrasound observation.

At this time, the user releases the above described fixing mechanism that fixes the insertion assisting instrument 31 and withdraws the insertion assisting instrument 31 from the insertion channel 12a of the insertion portion 12.

Subsequently, the user insertedly disposes a telescope or an endoscope or the like for the insertion channel 12a of the same insertion portion 12 into the same insertion channel 12a of the insertion portion 12 in place of the insertion assisting instrument 31. Here, for example, in the telescope or endoscope illustrated as one example in Fig. 21, an unshown protrusion group is provided that is the same as the protrusion for rotational direction positioning 31b and the engagement protrusions for fixing 31 c of the insertion assisting instrument 31. The telescope or the like is fixed in a similar manner as the insertion assisting instrument 31 by the fixing mechanism to one part of the grasping portion 13. Thereupon, vertical and horizontal directions of an optical image observed from an unshown optical observation window of the telescope, and an ultrasound transmitting/receiving direction of the ultrasound observation unit with respect to a protruding direction of a biopsy treatment instrument (unshown) such as a puncture needle are unambiguously fixed and the puncture needle can be visually recognized within the field of view of the ultrasound image. Next, while performing ultrasound observation, the user holds the grasping portion 13 and performs an operation to move the insertion portion 12 in an extraction direction. When the distal end portion 11, that is, the ultrasound transducer 21 comes to a portion in the vicinity of the prostate gland, the user temporarily stops the insertion/extraction of the ultrasound observation apparatus 1.

The user performs biopsy treatment while performing ultrasound observation, optical observation, endoscopic observation or the like using the above described biopsy treatment instrument (not shown) such as a telescope or an endoscope that has been inserted through the treatment instrument insertion channel. After the required biopsy is completed, the user withdraws the ultrasound probe 10 from the urethra to thereby complete the treatment.

As described above, according to the foregoing first embodiment, a configuration is adopted that takes into consideration excellent insertability into a conduit inside a body cavity such as the urethra that has a narrow blocked form as well as passage through a urethral bend portion, and therefore the distal end portion 11 is formed in a curved shape relative to the insertion portion 12 and a curvature angle thereof is set to approximately 20 to 35 degrees so that smoother insertability can be obtained.

Note that in the present embodiment a configuration is adopted in which the metal-made housing 23 and the insertion portion 12 that is composed of a metal member and the like are configured as separate members, and are configured so as to form an integrated structure when provided in a fixed condition with respect to each other using the fixing screw 24. In this case, although an example that uses the fixing screw 24 as fixing means for fixing the metal-made housing 23 and the insertion portion 12 with respect to each other is described in the above embodiment, the present invention is not limited thereto. For example, various other kinds of fixing means can be adopted, such as welding, adhesion, fixing by brazing, and fixing by soldering. In addition, as an alternative form, for example, a configuration may be adopted in which the metal-made housing 23 and the insertion portion 12 are integrally formed using a metal member or the like.

[Second Embodiment]

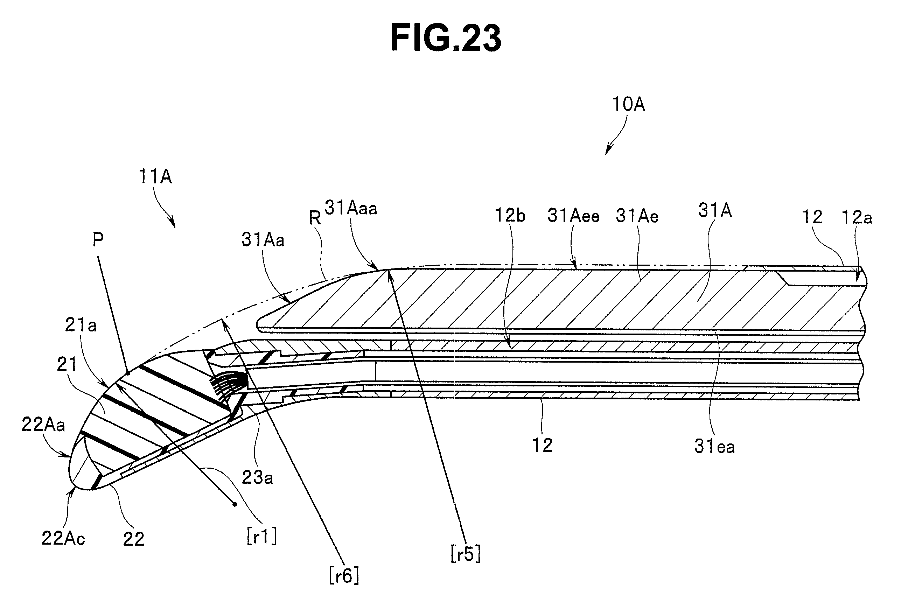

Next, an ultrasound observation apparatus according to a second embodiment of the present invention will be described. Fig. 23 is an enlarged longitudinal sectional view of a principal portion along a longitudinal direction of the ultrasound observation apparatus of the present embodiment, that is a sectional view that illustrates the internal configuration in the vicinity of the distal end portion of the ultrasound observation apparatus.

The present embodiment includes approximately the same configuration as the foregoing first embodiment, and differs slightly from the first embodiment only with respect to the outer shape from the insertion portion 12 to a distal end portion 11A. Accordingly, the configuration of a portion that differs relative to the above described first embodiment is described in detail hereunder, and diagrammatic representation and description of the same configuration as in the foregoing first embodiment is omitted.

The vicinity of the distal end portion 11A of an ultrasound observation apparatus 1A of the present embodiment is formed as shown in Fig. 23. That is, as shown in Fig. 23, a cross-sectional shape of the vicinity of an inclination starting point 31Aaa that is a portion that links a distal-end inclined face 31Aa of a distal-end shape portion 31Ae of the insertion assisting instrument 31A according to the present embodiment and a top face of the 31Aee of the distal-end shape portion 31Ae is formed in a relatively gentle arc shape that has a radius of curvature [r5]. Further, as shown in Fig. 23, the distal-end inclined face 31Aa and the top face 31Aee of the distal-end shape portion 31Ae smoothly connect at a face with the radius of curvature [r5] that includes the inclination starting point 31Aaa of the insertion assisting instrument 31 A.

That is, when a radius of curvature in the vicinity of the inclination starting point 31Aaa of the insertion assisting instrument 31A is taken as [r5] and a radius of curvature of the ultrasound transmitting/receiving face 21a of the ultrasound transducer 21 is taken as [r1], the arc shape in the vicinity of the inclination starting point 31Aaa and the arc shape of the ultrasound transmitting/receiving face 21a are formed so as to be included in the range of an arc R (arc having a radius of curvature [r6] indicated by a chain double-dashed line in Fig. 23) that contacts both the above described point of contact P and the inclination starting point 31Aaa. The remaining configuration is approximately the same as in the foregoing first embodiment.

According to the second embodiment that is configured as described above also, similarly to the foregoing first embodiment, smooth insertability can be achieved.

[Third Embodiment]

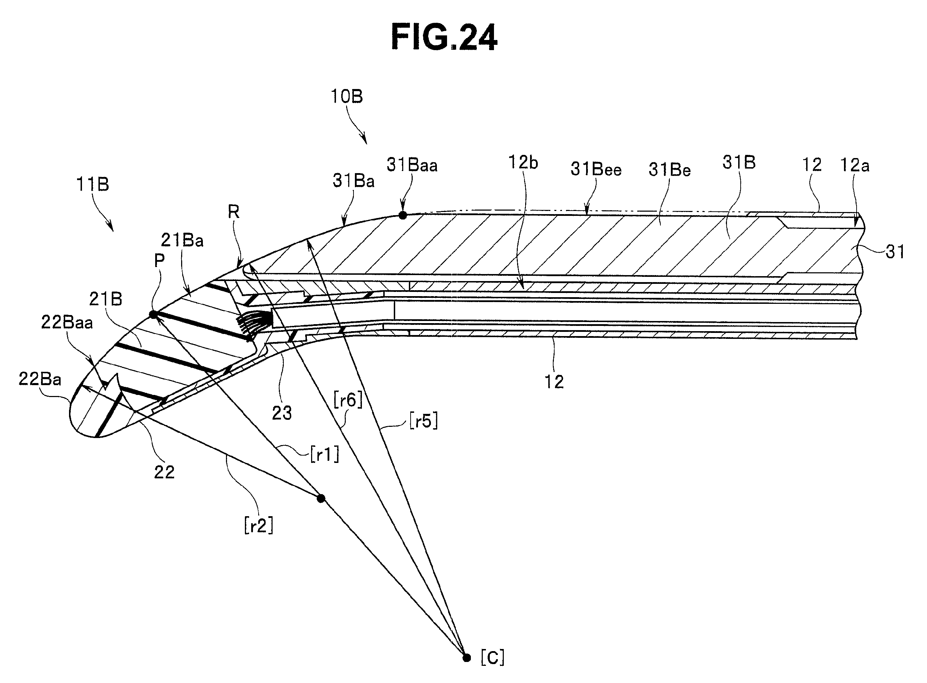

Next, an ultrasound observation apparatus according to a third embodiment of the present invention will be described. Fig. 24 is an enlarged longitudinal sectional view of a principal portion along a longitudinal direction of the ultrasound observation apparatus of the present embodiment, that is a sectional view that illustrates the internal configuration in the vicinity of the distal end portion of the ultrasound observation apparatus.

The present embodiment includes approximately the same configuration as the foregoing first and second embodiments, and differs slightly therefrom only with respect to the outer shape from the insertion portion 12 to the distal end portion 11. Accordingly, the configuration of a portion that differs relative to the above described first and second embodiments is described in detail hereunder, and diagrammatic representation and description of the same configuration as in the foregoing embodiments is omitted.

The vicinity of a distal end portion 11B of an ultrasound observation apparatus 1B of the present embodiment is formed as shown in Fig. 24. That is, in the present embodiment,
a radius of curvature in the vicinity of an inclination starting point 31 Baa of the insertion assisting instrument 31B is referred to as "[r5]";
a radius of curvature of the arc R that contacts both the point of contact P and the inclination starting point 31Baa is referred to as "[r6]";

a radius of curvature of an ultrasound transmitting/receiving face 21 Ba of an ultrasound transducer 21B is referred to as "[r1]"; and
a radius of curvature of a distal end continuation portion 22Ba of the ultrasound transducer 21B is referred to as "[r2]".

In this case, the distal end portion 11B and the insertion assisting instrument 31B are formed so that the relationship between the above described radii of curvature [r1], [r5], and [r6] is:

$$[r1] \approx [r5] \approx [r6].$$

The remaining configuration is substantially the same as in the foregoing first embodiment.

According to the third embodiment that is configured as described above also, similarly to the foregoing first and second embodiments, smooth insertability can be achieved.

Note that the present invention is not limited to the above described embodiments, and naturally various modifications and applications can be implemented within a range that does not deviate from the spirit and scope of the present invention. Further, the above described embodiments include inventions of various stages, and various inventions can be extracted by appropriately combining a plurality of the disclosed configuration requirements. For example, if a problem to be solved by the invention can be solved and the effects of the invention are obtained even after omitting some of the configuration requirements from the entire configuration requirements shown in the respective embodiments described above, then the configuration obtained by omitting the configuration requirements can be extracted as an invention.

The present invention can be applied not just to an endoscope control apparatus in the medical field, but also to an endoscope control apparatus in the industrial field.

The present application is filed claiming the priority of Japanese Patent Application No. 2011-236391 filed in Japan on October 27, 2011.

The contents disclosed in the above basic application are incorporated by reference in the present description, claims and drawings.

Industrial Applicability

The present invention is applicable not only to an endoscope control apparatus in a medical field but also to an endoscope control apparatus in an industrial field.

**Claims**

1. An ultrasound observation apparatus, comprising:

   an ultrasound transducer having an ultrasound transmitting/receiving face that transmits/receives ultrasound;
   a holding part that fixedly holds the ultrasound transducer;
   an insertion portion formed in a rigid cylindrical shape in which the holding part is fixedly installed at a distal end; and
   an insertion assisting instrument that is formed in a rigid rod shape or cylindrical shape, and that is insertedly disposed in a freely insertable/extractable manner in the insertion portion;
   wherein:

   the holding part is fixedly installed at the distal end of the insertion portion so as to form a shape that is curved with a predetermined angle in a separating direction with respect to a longitudinal direction from the distal end of the insertion portion; and
   a curvature angle of the holding part with respect to the longitudinal direction of the insertion portion is set so that, when the insertion assisting instrument is insertedly disposed in the insertion portion, an extension line of a straight line that contacts a distal end face of the insertion assisting instrument contacts the ultrasound transmitting/receiving face.

2. The ultrasound observation apparatus according to claim 1, wherein:

   the distal end face of the insertion assisting instrument is formed by an inclined flat face or an inclined cylindrical face; and
   the curvature angle of the holding part with respect to the longitudinal direction of the insertion portion is set so that, when the insertion assisting instrument is insertedly disposed in the insertion portion, an extension line of a straight line along the inclined flat face of the insertion assisting instrument on a sectional view including a longitudinal axis contacts the ultrasound transmitting/receiving face.

3. The ultrasound observation apparatus according to claim 2, wherein:

   a back face of the holding part is formed by a flat face or a cylindrical face, and on a sectional view which includes a longitudinal axis,
   the curvature angle of the holding part with respect to the longitudinal direction of the insertion portion is set so that a straight line along the back face of the holding part and a straight line along the inclined flat face of the insertion assisting instrument are parallel or intersect on a distal end side at an acute angle.

4. The ultrasound observation apparatus according to claim 1, wherein:

the distal end face of the insertion assisting instrument is formed into an arc shape on a sectional view which includes a longitudinal axis, and

an arc along the distal end face of the insertion assisting instrument and an arc of the ultrasound transmitting/receiving face are formed to be a continuous curve.

5. The ultrasound observation apparatus according to claim 1, wherein:

on the ultrasound transmitting/receiving face, a distal end corner portion is formed in a rounded shape.

6. The ultrasound observation apparatus according to claim 1, wherein:

the insertion portion has an opening located at a predetermined region towards the distal end.

7. The ultrasound observation apparatus according to claim 1, wherein:

in a state in which the insertion assisting instrument is insertedly disposed in the insertion portion, in at least a predetermined region towards the distal end,

an outer circumferential length of a cross section that is perpendicular to a longitudinal direction of the holding part and the insertion portion is formed so that the circumferential length of a second portion that is towards a proximal end is larger than that of a first portion that is furthest towards the distal end.

8. The ultrasound observation apparatus according to claim 7, wherein:

the first portion is a portion in a vicinity of an approximately central part in the longitudinal direction of the holding part; and

the second portion is a portion in a vicinity of a proximal end side of the opening at the distal end of the insertion portion.

9. The ultrasound observation apparatus according to claim 7, wherein:

the cross-sectional outer circumferential length in the predetermined region towards the distal end changes so as to increase stepwise from a distal end side at least at four portions comprising the first portion and the second portion and a third portion and a fourth portion that are positioned between the first portion and the second portion.

10. The ultrasound observation apparatus according to claim 9, wherein:

the third portion is a portion in a vicinity of an approximately central part of a distal-end inclined face of the insertion assisting instrument; and

the fourth portion is a portion in a vicinity of an approximately central part of the opening at the distal end of the insertion portion.

11. The ultrasound observation apparatus according to claim 7, wherein:

the cross-sectional outer circumferential length in the predetermined region towards the distal end changes so as to gradually increase from the first portion to the second portion.

12. The ultrasound observation apparatus according to claim 1, wherein:

the insertion assisting instrument comprises a treatment instrument insertion path.

13. The ultrasound observation apparatus according to claim 1, wherein:

the insertion assisting instrument comprises an optical observation portion.

14. The ultrasound observation apparatus according to claim 1, wherein:

the holding part is formed by a member made of resin that has electrical insulation properties; and

an outer surface of the holding part is covered by a housing member made of metallic material.

15. The ultrasound observation apparatus according to claim 1, wherein:

the housing member and the insertion portion are constituted by two members.

# FIG.1

EP 2 671 513 A1

EP 2 671 513 A1

# FIG.2

# FIG.3

# FIG.4

EP 2 671 513 A1

**FIG.5**

FIG.6

# FIG.7

EP 2 671 513 A1

# FIG.8

EP 2 671 513 A1

# FIG.9

FIG.10

# FIG.11

# FIG.12

FIG.13

# FIG.14

31

12

22

23

21

# FIG.15

31

12

23

25c

25a    25b

# FIG.16

31

12

25c

25a   25b

# FIG.17

31

12

25c

25a   25b

# FIG.18

# FIG.19

# FIG.20

# FIG.21

# FIG.22

# FIG.23

EP 2 671 513 A1

# FIG.24

EP 2 671 513 A1

# FIG.25

EP 2 671 513 A1

EP 2 671 513 A1

# FIG.26

31

12

23

25c

25a   25b

37

| INTERNATIONAL SEARCH REPORT | International application No. |
|---|---|
| | PCT/JP2012/077574 |

A. CLASSIFICATION OF SUBJECT MATTER
*A61B8/12*(2006.01)i, *A61B1/00*(2006.01)i, *A61B1/303*(2006.01)i, *A61B1/307*
(2006.01)i, *A61B1/31*(2006.01)i

According to International Patent Classification (IPC) or to both national classification and IPC

B. FIELDS SEARCHED

Minimum documentation searched (classification system followed by classification symbols)
A61B8/12, A61B1/00, A61B1/303, A61B1/307, A61B1/31

Documentation searched other than minimum documentation to the extent that such documents are included in the fields searched
Jitsuyo Shinan Koho        1922-1996   Jitsuyo Shinan Toroku Koho    1996-2012
Kokai Jitsuyo Shinan Koho   1971-2012   Toroku Jitsuyo Shinan Koho    1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2000-185044 A (Asahi Optical Co., Ltd.), 04 July 2000 (04.07.2000), fig. 4 & US 6338717 B1         & DE 19962209 A & DE 19962209 A1 | 1-15 |
| A | JP 9-503942 A (FemRx Inc.), 22 April 1997 (22.04.1997), fig. 3 & US 5456689 A          & US 5527331 A & EP 723422 A           & WO 1995/010981 A1 & AU 7977794 A | 1-15 |
| A | JP 8-107900 A (Astem Tech Co., Ltd.), 30 April 1996 (30.04.1996), fig. 7 & US 5685824 A | 1-15 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 26 November, 2012 (26.11.12) | 04 December, 2012 (04.12.12) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2012/077574 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2005-319313 A  (Mayo Foundation for Medical Education and Research), 17 November 2005 (17.11.2005), fig. 1, 2, 5 to 9 & US 5325860 A          & EP 611292 A & WO 1993/008738 A1      & DE 69233494 D & SG 50556 A            & CA 2121353 A & AT 291373 T            & ES 2241210 T | 1-15 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001037775 A
- JP 2011236391 A